# EUROPEAN PATENT APPLICATION

(11) **EP 0 811 361 A1**
(43) Date of publication of application: **10.12.1997**
(21) Application number: 96108736.8
(22) Date of filing: 03.06.1996
(51) Int. Cl.: A61F 13/72

(54) **Garment for use with a disposable absorbent article**

(71) Applicant: THE PROCTER & GAMBLE COMPANY, Cincinnati, Ohio 45202 (US)
(72) Inventor: Willms, Eric-Joachim, 65812 Bad Soden (DE)
(74) Representative: Canonici, Jean-Jacques

(57) **Abstract**

A garment 10 for holding a disposable absorbent article 21 comprising a front section 11; a rear section 12; a crotch section 13 disposed between the front section 11 and the rear section 12; and a pair of elasticised leg openings 14; characterised in that the garment 10 comprises elasticised pressure zones 15 to create at least one pocket 16 in the front section 11 or the rear section 12; the pocket 15 being located inboard of the elasticised leg openings 14.

## Description

### Field of the invention

The invention relates to a garment for use in conjunction with a disposable absorbent article. The garment is designed to have a superior contact area with the body of the wearer and is primarily directed to incontinence sufferers.

### Description of the prior art

Garments that protect against incontinence are well known in the art. These garments are designed to receive and contain urine, faeces and other bodily discharged materials and to isolate these discharges both from the body and from the surroundings of the wearer. Typically, these garments have a portion that is designed to hold or position a disposable absorbent article against the body of the wearer. A number of these garments also have structural features that hold the absorbent article in a desired position within the portion of the garment until the absorbent article is soiled and discarded.

Prior art developments include GB 2 282 053, which describes a panty that may be used by men who need to wear an absorbent product in the region of their groin as a result of a medical condition. The panty is so configured and constructed that, in use, the absorbent product firmly embraces the body of the wearer by the panty via a partial lining of impermeable material, and movement of the absorbent product is prevented. This solution has a degree of effect, but problems still arise with regard to tightness which may cause wearer discomfort, rolling up of the leg areas leading to leakage of fluids, and wearer movement can create gapping in the groin and back regions.
GB 2 185 678 A discloses a disposable undergarment comprising an integral absorbent pad that can function as a light incontinence garment. The absorbent pad stretches upwards from the crotch region both in the back and the front region to a point higher than normal absorbent pads. The device is designed to substantially minimise the leakage of fluids in overnight use. The configuration may lead to wearer discomfort due to the built-in and high positioning of the absorbent pad.
US 4,355,425 describes an improved panty and method of making same that has both non-woven porous fabric panels and non-woven elastic members. The panty is characterised by elastication in all directions. Problems may arise regarding body contact and the positioning of the absorbent product. Furthermore, the panty is only designed for use by a woman or a child.
WO 92/00051 details an undergarment that includes a permanently stretched region within which the incontinence guard is placed and in which the material has a lower elasticity than in the remaining regions of the undergarment. Such features enable the correct and ready positioning of the incontinence guard and improved wearer confidence. Nevertheless, the configuration leads to bunching in the back region and does not guarantee a high body contact. Furthermore, when the incontinence guard is loaded, the undergarment is incapable of covering the incontinence guard effectively and leakage may occur.
WO 95/09594 relates to a light incontinence panty that is characterised by elastic devices, which extend from the front to the back part of the panty. The elastic devices may comprise elastic threads, ribbons or bands that are preferably mounted between two layers or sheets comprising the panty. The invention however does not disclose a garment that incorporates elasticity through an integral knitting technology. The object of the panty design is to both press the absorbent article into close contact with the body of the wearer and to form the absorbent article into a shape that conforms to the anatomy of the wearer. The panty may suffer from such drawbacks as wearer discomfort, sagging of the waistband and a poor fit.

As is evident, the prior art garments typically reveal many shortcomings, namely poor body contact due to the movement of the wearer, poor fit when loaded and dry, lack of wearer comfort, increased bulkiness leading to a clumsy and unappealing appearance, and leakage of fluids accompanied by the spoilage of outer garments and bedding.

It has now been discovered that the above drawbacks can be alleviated by a garment as disclosed in the present invention. The garment of the present invention enables both a superior overall body contact and an excellent fit when the disposable absorbent article is both wet and dry to be obtained. This subsequently leads to several benefits such as better positioning and containment of the absorbent article, improved acquisition properties, reduction in leakage and superior wearer confidence and comfort.

Furthermore, the garment of the present invention permits the above benefits to be achieved with the use of disposable absorbent articles that are of any size or shape.

### Object and summary of the invention

The object of the present invention is to provide a garment capable of holding a disposable absorbent article which results in a superior overall body contact and an excellent fit when the absorbent article is both wet and dry and independent of whether or not the wearer is stationary or moving.
In accordance with the object of the invention, a garment for holding a disposable absorbent article is provided. The garment comprises a front section, a rear section, a crotch section between the front section and the rear section and a pair of elasticised leg openings, characterised in that the garment comprises elasticised pressure zones which result in the creation of at least one pocket in either the front section or the rear section. The created pocket or pockets is/are located inboard of the leg elasticised openings and the elasticised pressure zones are preferably adjacent to at least a portion of the elasticised leg openings. An additional elasticised pressure zone is preferably present in the area of the front section or the rear section corresponding to the created pocket; said additional elasticised pressure zone being preferably centred around the longitudinal axis of the garment. In a preferred embodiment of the invention, the elasticised pressure zones and the additional elasticised pressure zone present in the area corresponding to the pocket are located in the front section and most preferably an elasticised pressure zone is present in the rear section. The zones are preferably in the form of either a continuous or an interrupted pattern and are integrally knit into the front section, rear section and crotch section.
It is a preferred feature of the invention that none of the elasticised pressure zones forms a connection with the waistband of the garment.
The front section and the rear section of the garment preferably comprise a mixture of nylon fibres, cotton and Lycra® and the crotch section and the elasticised pressure zones comprise a mixture of nylon fibres, cotton and a higher density of Lycra®. In addition, the front section, the rear section, the crotch section and the elasticised pressure zones are preferably treated with liquid resistant agents.
The garment of the present invention can comprise seams that are formed by joining the front section to the rear section, but the garment can also constitute a single piece of material, i.e., it can be seamless. The seams, when present, are preferably disposed on the outer surface of the garment. In the preferred embodiment of the invention, the crotch section is seamless.
The garment is typically overall less stretchable in a direction longitudinal to the wearer and more stretchable in a direction transverse to the wearer. The crotch section is preferably elastically extensible both in the longitudinal and transverse directions, though the preferred embodiment is endowed with a lower elastic extensibility in the longitudinal direction and a higher elastic extensibility in the transverse direction.
According to the present invention, the object is achieved by a garment having the characteristics specified in the claims.

### Brief description of the drawings

The invention will be described hereinafter with reference to the following drawings:
Figure 1 shows a front view of a preferred embodiment of the present invention;
Figure 2 is a rear view of a more preferred embodiment of the invention; the front section corresponding to that which is shown in Figure 1;
Figure 3 is a side view of the more preferred embodiment of the invention; the front section and the rear section corresponding to that which is shown in Figures 1 and 2 respectively;
Figure 4 shows, in a composite fashion and on a magnified scale, the loop structure of the preferred tubular knit fabric of the garment.

### Detailed description of the invention

As used herein, the term "garment" refers to an article of clothing that is worn in proximity to the body of the wearer. As used herein, the term "disposable" refers to structures which are not intended to be laundered or otherwise restored or reused after use (i.e., they are intended to be discarded after a single use and preferably to be recycled, composted or otherwise disposed of in an environmentally friendly manner). As used herein, the term "absorbent article" describes a product which can be worn by both males and females adjacent to their genitalia for the purposes of absorbing and containing urine, faeces and other bodily discharged materials. Absorbent articles for use in the garment herein are preferably inserts designed to cope with incontinence problems. It should be understood however that the present invention also applies to other absorbent articles such as baby diaper inserts, interlabial devices, sanitary napkins, panty liners and the like. As used herein, the term "elasticised pressure zones" refers to pressure areas present in the garment at selected locations, that are more elasticised than the front and rear section of the garment and in which elastic forces act, to ensure that the absorbent article is kept in place and pressed closely to the body of the wearer. As used herein, the term "pocket" is the result of an interaction between the elasticised pressure zones and the other sections of the garment, i.e., the front section, rear section and crotch section and the elasticised leg openings, which leads to the formation of a pouch. As used herein, the term "inboard" means inside the elasticised leg openings.
Even though the garment of the present invention is designed for use in conjunction with a disposable absorbent article, it is also foreseen that the garment may be worn without any absorbent article. The garment herein is preferably designed for re-use and thus can be laundered. Nevertheless, by altering the inherent material composition of the garment, the garment can be made disposable.

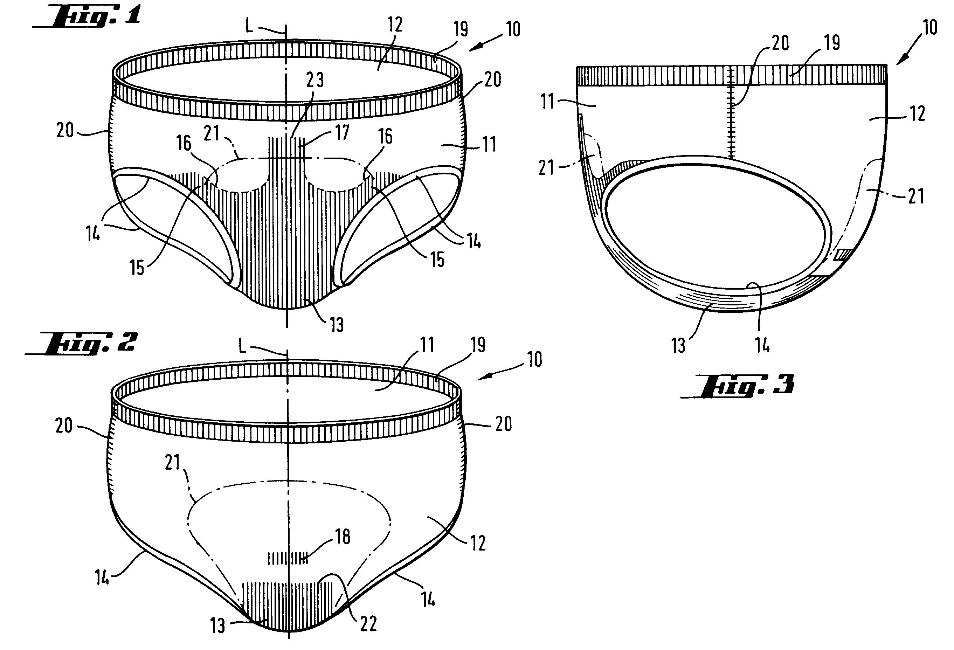

In a preferred embodiment of the invention as shown in Figure 1, the elasticised pressure zones 15 and the additional elasticised pressure zone 17 are located in the front section 11 and as shown in a more preferred embodiment of the invention in Figure 2, an elasticised pressure zone 18 is present in the rear section 12. The additional elasticised pressure zone 17 can vary lengthwise from 10 millimetres to 450 millimetres and widthwise from 10 millimetres to 500 millimetres. The elasticised pressure zones 15 can begin at the intersection of the elasticised leg openings 14 and the side seams 20, although for the preferred embodiment, the elasticised pressure zones 15 are disposed adjacent to at least a portion of the elasticised leg openings. As used herein, the term "adjacent" means to lie near and as such encompasses the range varying from 0 millimetres to 50 millimetres. In the preferred embodiment of the invention, the elasticised pressure zones 15 in the front section 11 are preferably abutting on the elasticised leg openings 14. If the elasticised pressure zones 15 are located in the rear section, abutment need not take place and the zones 15 may be disposed a certain distance, namely 5 millimetres to 50 millimetres, from the elasticised leg openings 14. The elasticised pressure zone 18 lies parallel to the periphery 22 of the rear section 13. The periphery 22 of the rear section 13 is the point at which a transition occurs in the integral knitting pattern of the garment 10. The dimensions of the elasticised pressure zone 18 can vary from 5 millimetres to 500 millimetres. The shape of the zone 18 is preferably rectangular although the zone 18 can take on any shape. All of the elasticised pressure zones 15, 17, 18 form no connection with the waistband 19. In a preferred embodiment, a distance varying from 5 millimetres to 200 millimetres should be left free between the upper periphery 23 of the elasticised pressure zone 17 and the waistband 19.
Elastic forces act in the elasticised pressure zones 15, 17, 18. In the case of the preferred embodiment, the forces exerted in the elasticised pressure zone 15 act parallel to the leg cut and move the leg elastics inwards towards the body of the wearer to create at least one pocket 16 in the front section 11 and a recess in the crotch section 13. The additional elasticised pressure zone 17, which acts as a central pressure point, allows for the formation of two pockets 16; one on each side of the elasticised pressure zone 17. The presence of the pockets 16 is critical for the use of the garment 10 with disposable absorbent articles 21, which are designed for severe cases of incontinence. The pockets 16 enable the disposable absorbent article 21 to be fitted securely, comfortably and without the risk of displacement even when heavily loaded. In the case of disposable absorbent articles designed for light to moderate conditions of incontinence, the presence of the additional elasticised pressure zone 17 is not essential. This can be explained by the fact that the disposable absorbent articles 21 are of a different design, namely, of a shorter length and of a different core shape. The elasticised pressure zone 18 located in the rear section 12 of the garment 10 prevents bunching of the disposable absorbent article 21. The effect of all the elasticised pressure zones 15, 17, 18 is to tighten up the garment 10, reduce dramatically the presence of gaps or low pressure zones, and to allow for a more comfortable fit and a superior contact area with the body of the wearer.
In the preferred embodiment as drawn in Figure 3, the leg cut typically ranges from 100 millimetres to 350 millimetres. The cut of the leg openings is designed to direct the contractive force of the leg elastics in a more preferred direction than would be possible if a more circular shape were used. The contractive force should not be so high as to cause discomfort to the wearer. A contractive force of at least 0.19 newtons is necessary to minimise the risk of gapping around the periphery of the leg openings. Preferably, the contractive force should be at least about 0.38 newtons and more preferably, about 0.78 newtons. The elongation for the leg elastics is 170 percent ± 10 percent. The leg elastics can be joined to the front section 11, the rear section 12 and the crotch section 13 about the periphery of the leg openings using means known in the art. For example, the leg elastics can be joined to the front section 11, the rear section 12 and the crotch section 13 using adhesive means or by mechanical means such as stitching. For the preferred embodiment, the leg elastics are preferably joined to the front section 11, the rear section 12 and the crotch section 13 by stitching.
In the preferred embodiment as drawn in Figures 1 and 2, the elasticised pressure zones 15 extend from an area on the front section 11 adjacent to the elasticised leg openings 14 to an area of the crotch section 13. These elasticised pressure zones 15 are integrally knit into the crotch section 13. The crotch section 13 is preferably seamless to prevent the loss of elastication that would be caused by the presence of a seam. The crotch section 13 covers an area stretching from the periphery 22 of the rear section 12 to an area abutting on a portion of the elasticised leg openings 14 and the additional elasticised pressure zone 17. All these zones 15, 17 including the elasticised zone 18 are integrally knit into the front section 11, the rear section 12 and the crotch section 13 of the garment 10 and are designed to hold the disposable absorbent article 21 both when wet and dry in a close and superior fitting position to the body of the wearer.

The garment 10 may comprise woven, non-woven or knit fabrics having suitable feel, appearance and surface properties. In the preferred embodiment of the invention, the garment 10 comprises a knit fabric, which provides a comfortable tactile feel. A particularly preferred knitting means involves first knitting a seamless tubular blank approximately half the final width of the garment 10. The tubular blank may be knit to have an hour glass shape so as to provide for the leg openings in the garment 10 or alternatively, portions of the opened tube may be cut away to provide for such leg openings. The resultant garment 10 is then completely seamless. The preferred knitting means is described in greater detail in US 3,985,004, the disclosure of which is incorporated herein by reference.

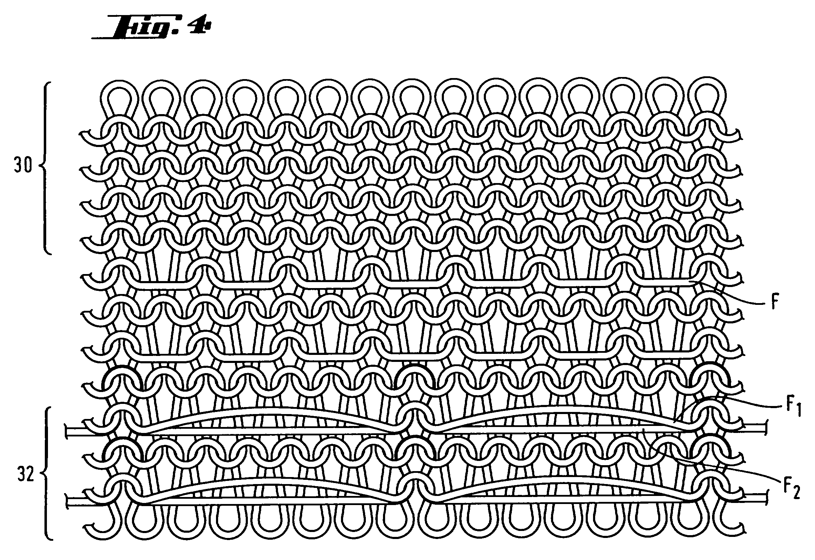

Figure 4 shows, in a composite fashion and on a magnified scale, the loop structure of the preferred tubular knit fabric of the garment 10. The knit pattern of the courses shown as 30 corresponds to the knit pattern used for the front section 11 and the rear section 12. The courses shown as 32 correspond to the knit pattern used for the crotch section 13 and the elasticised pressure zones 15, 17, 18. The floats, shown as F, F1 and F2 help provide the crotch section 13 and the elasticised pressure zones 15, 17, 18 with an attractive ribbed appearance. These ribs also help direct the flow of any bodily discharged materials that may be deposited on the crotch section 13 and the elasticised pressure zones 15, 17, 18 in a direction parallel to the longitudinal axis L of the garment 10. Furthermore, the elasticised pressure zones 15, 17, 18 are in the form of either a continuous or an interrupted pattern. If the elasticised pressure zones 15, 17, 18 are in the form of an interrupted pattern, then the pattern can be modelled in such a way as to correspond to pressure points that conform to the shape of the disposable absorbent article 21. In other words, glue points could be positioned on the backsheet of the absorbent article 21 which could then be adhesively attached to pre-marked points on the garment 10. This would ensure the excellent positioning of the disposable absorbent article 21 in the garment 10.
The blank for the garment 10 is first knit in a tubular form. The tubular blank is then slit walewise and opened to form a flat blank for the garment 10. The blank for the garment has end edges and side edges. The elastic waistband 16 may be formed by providing an elastic member adjacent to each distal end of a garment blank, C-folding each distal end of the blank about itself to form end edges, and seaming the distal ends to the front section 11 and the rear section 12 to form the elastic waistband 16. The elastic waistband 16, however, preferably comprises a turned wet. The leg elastics are joined to that portion of the side edges which will surround the leg openings. The blank for the garment 10 is then folded about the transverse centreline and opposing portions of the side edges that lie between the leg opening and the end edge are joined to form the side seams 20 completing the assembly of the garment 10.

In order to improve the breathability properties so the garment 10 is more comfortable to wear, the front section 11 and the rear section 12 preferably comprise a mixture of nylon fibres, cotton and Lycra® and the crotch section 13 and the elasticised pressure zones 15, 17, 18 preferably comprise a mixture of nylon fibres, cotton and a higher density of Lycra® and the elasticised leg openings 14 preferably comprise a similar mix. The garment 10 may also be constructed from other synthetic fibres such as perlon, polypropylene, elastomeric staple integrated composites, block copolymers of polystyrene, polyisoprene or polybutadiene, copolymers of ethylene, natural rubbers, urethanes, Kratons and coextrusions/blends of the afore-mentioned.
The combination of the preferred materials results in a garment 10 with varying degrees of elastic extensibility, namely, the front section 11 and rear section 12 have the lowest degree of elastic extensibility in comparison to the crotch section 13, the elasticised pressure zones 15, 17, 18 and the elasticised leg openings 14, and the elasticised leg openings 14 exhibit the highest degree of elastic extensibility. The crotch section 13 is elastically extensible both in the longitudinal and transverse directions, though the preferred embodiment is endowed with a lower elastic extensibility in the longitudinal direction and a higher elastic extensibility in the transverse direction. This property of elastic extensibility enables the garment 10 to fit a variety of shapes and sizes and provides a superior contact area with the body of the wearer. The front section 11 and the rear section 12 are preferably so constructed as to have a stretch modulus in the range from 51 g/cm to 63 g/cm, more preferably in the range from 55 g/cm to 59 g/cm; the crotch section 13 and the elasticised pressure zones 15, 17, 18 are preferably so constructed as to have a stretch modulus which is higher than the stretch modulus value for the front section 11 and the rear section 12 and which is equal to or less than about 118 g/cm, more preferably equal to or less than 71 g/cm; and the elasticised leg openings 14 are preferably so constructed to have a stretch modulus which is higher than the stretch modulus values for the front section 11, the rear section 12, the crotch section 13 and the elasticised pressure zones 15, 17, 18 and is in the range from 213 g/cm to 232 g/cm, more preferably in the range 216 g/cm to 228 g/cm. A suitable method for measuring the stretch modulus is described below in the "Test Methods" section.
To provide liquid resistance to the preferred embodiment of the garment 10, the front section 11, the rear section 12 and the crotch section 14 and the elasticised pressure zones 15, 17, 18 are treated with liquid resistant agents. In particular for the preferred embodiment, the garment 10 can be treated with materials including fatty acid or fatty alcohol derivatives, silicones, polyurethanes and fluorocarbons. Fluorocarbon materials are preferred because of the low surface energy of the fluorocarbon surfaces. A suitable process for treating the garment 10 is as follows: (1) the blank is knitted as described above; (2) the blank is washed to remove the fibre finishes and the like; (3) the washed blank is treated with the fluorocarbon material as the last step of the wash cycle according to the instructions of the material supplier; (4) the blank is allowed to dry; (5) the blank is slit and (6) the slit blank is converted into a finished garment as described above. The method treats the entire garment blank with the fluorocarbon material. Alternatively, the fluorocarbon material can be selectively applied to only the front section 11 and the rear section 12 using means such as padding, spraying or other means familiar to the art to apply a solution (or emulsion or suspension). Liquid resistance can also be provided by means of a fluid resistant film laminate.

Any size or shape of absorbent article 21 can be used with the garment 10 in order to derive the benefits described herein. It has been found that a particularly preferred absorbent article 21 for use in adult incontinence protection has a length ranging from 400-700 millimetres and a width ranging from 110-300 millimetres. The particularly preferred article 21 has an hour-glass shape. The garment 10 is capable of holding an absorbent article 21 weighing up to 2000 grams when loaded if the absorbent article 21 is designed with the afore-mentioned dimensions.
The crotch width of the garment 10 can vary from 60-200 millimetres and the overall length of the garment 10 can span a range from 200 millimetres to 500 millimetres in the relaxed state. Garments 10 made according to the preferred embodiment of the invention can conveniently be made in small, medium and large sizes to accommodate the proportions of a wide number of consumers. While it is to be appreciated that the invention is not limited to embodiments having these dimensions, it has been found that these three garment sizes will accommodate a selection of wearers ranging from adolescents to rather large adults.

The garment 10 is used by removing any release liner that may be provided on the disposable absorbent article 21 and thereafter by placing the absorbent article 21 in the garment 10 using sufficient pressure to insure that the absorbent article 21 is attached in a secure manner. If the absorbent article 21 is furnished with flaps, the absorbent article 21 is positioned such that the flaps drape over the edge of the elasticised leg openings 14 such that the flap attachment means are placed in contact with the outer surface of the crotch section 13. The wearer can then draw on the garment 10 in the conventional manner. When the wearer is through wearing the garment 10, the absorbent article 21 is removed and the garment 10 is laundered and ready for re-use.

### Test Methods

### Stretch modulus and elastic contractions

This method is intended to qualify a force comparable to the force exerted on the body of the wearer by elastically extensible materials that may be used in a garment over an extension range comparable to that seen in the wear cycle of a garment.

### Set-up conditions

The method described in INDA (Association of Nonwoven Fabric Industry) Standard Test 110.1-92 is suitable and the following set-up conditions are used:
- Gauge length:: 5.08 cm
- Crosshead speed:: 25.4 cm/minute
- Tensile testing machine:: Appropriate for expected force range Model 5564, available from Instron Corp., Canton, MA (USA) is suitable.
- Sample width:: 2.54 cm. For samples less than 2.54 cm wide, measure the sample width and adjust the measured force by the ratio of 2.54 cm to the measured width.
- Sample size:: At least three samples per material
tested.

### Calculations

Force₂₅: Force at 25 % elongation (g/cm)
Force₅₀: Force at 50 % elongation (g/cm)
Elastic contraction = Force₂₅
Stretch modulus = (Force₅₀ - Force₂₅)/0.25

The mean and standard deviation for the stretch modulus, and for the elastic contractions (leg elastics only) should be reported.

### Glossary

- Garment: 10
- Front section: 11
- Rear section: 12
- Crotch section: 13
- Elasticised leg openings: 14
- Elasticised pressure zone: 15
- Pocket: 16
- Additional elasticised pressure zone: 17
- Elasticised pressure zone in rear section: 18
- Waistband: 19
- Side seams: 20
- Disposable absorbent article: 21
- Periphery of rear section: 22
- Upper periphery of additional elasticised pressure zone: 23
- Knit pattern for front section and rear section: 30
- Knit pattern for crotch section and elasticised pressure zones: 32
- Floats: F, F1, F2
- Longitudinal axis of garment: L

## Claims

1. A garment 10 for holding a disposable absorbent article 21 comprising
a front section 11; a rear section 12; a crotch section 13 between said front section 11 and said rear section 12; and a pair of elasticised leg openings 14;
characterised in that
said garment 10 comprises elasticised pressure zones 15 to create at least one pocket 16 in said front section 11 or said rear section 12, said pocket 15 being located inboard of said elasticised leg openings 14.

2. A garment 10 according to claim 1 wherein said elasticised pressure zones 15 are adjacent at least a portion of said elasticised leg openings 14.

3. A garment 10 according to any of the previous claims wherein an additional elasticised pressure zone 17 is present in the area of said front section 11 or said rear section 12 corresponding to said pocket 16.

4. A garment 10 according to any of the previous claims wherein said additional elasticised pressure zone 17 is centred around the longitudinal axis L of said garment 10.

5. A garment 10 according to any of the previous claims wherein said elasticised pressure zones 15, 17 are located in said front section 11.

6. A garment 10 according to claim 5 wherein a further elasticised pressure zone 18 is present in said rear section 12.

7. A garment 10 according to any of the previous claims wherein said garment 10 has a waistband 19 and all of said elasticised pressure zones 15, 17, 18 form no connection with said waistband 19.

8. A garment 10 according to any of the previous claims wherein said elasticised pressure zones 15, 17, 18 are in the form of either a continuous or an interrupted pattern.

9. A garment 10 according to any of the previous claims wherein said elasticised pressure zones 15, 17, 18 are integrally knit into said front section 11, said rear section 12 and said crotch section 13.

10. A garment 10 according to any of the previous claims wherein said front section 11 and said rear section 12 comprise a mixture of nylon fibres, cotton and Lycra®, and said crotch section 13 and said elasticised pressure zones 15, 17, 18 comprise a mixture of nylon fibres, cotton and a higher density of Lycra®.

11. A garment 10 according to any of the previous claims wherein said front section 11, said rear section 12, said crotch section 13 and said elasticised pressure zones 15, 17, 18 are treated with liquid resistant agents to provide liquid resistance.

12. A garment 10 according to any of the previous claims wherein said front section 11 and said rear section 12 are joined by side seams 20; said side seams 20 being disposed on the outer surface.

13. A garment 10 according to any of the previous claims wherein said garment 10 is less stretchable in a direction longitudinal to the wearer and more stretchable in a direction transverse to the wearer.

14. A garment 10 according to any of the previous claims wherein said crotch section 13 is elastically extensible both in the longitudinal and transverse direction, more preferably with a higher elastic extensibility in said direction transverse to the wearer and a lower elastic extensibility in said direction longitudinal to the wearer.

15. A garment 10 according to any of the previous claims wherein said front section 11 and said rear section 12 have a stretch modulus in the range from 51 g/cm to 63 g/cm; said crotch section 13 and said elasticised pressure zones 15, 17, 18 have a stretch modulus which is equal to or less than about 118 g/cm and which is higher than said stretch modulus value for said front section 11 and said rear section 12; and said elasticised leg openings 14 have a stretch modulus in the range from 213 g/cm to 232 g/cm which is higher than said stretch modulus values for said front section 11, said rear section 12, said crotch section 13 and said elasticised pressure zones 15, 17, 18.
